# EUROPÄISCHE PATENTSCHRIFT

(11) **EP 0 871 384 B1**
(45) Veröffentlichungstag und Bekanntmachung des Hinweises auf die Patenterteilung: **26.07.2000**
(21) Anmeldenummer: 96943874.6
(22) Anmeldetag: 06.12.1996
(51) Int. Cl.: A47G 9/00, A47C 27/00, A62B 18/00

(54) **RUHE- UND ENTSPANNUNGSHILFSMITTEL**
CALMING AND RELAXING OBJECT
OBJET APAISANT ET RELAXANT

(30) Priorität: 07.12.1995 DE 29519434 U; 26.01.1996 DE 19602825; 31.05.1996 DE 19622046
(43) Veröffentlichungstag der Anmeldung: 21.10.1998
(73) Patentinhaber: Frenzel, Detlef, 84072 Au/Hallertau (DE)
(72) Erfinder: Frenzel, Detlef, 84072 Au/Hallertau (DE)
(74) Vertreter: Winter, Brandl, Fürniss, Hübner, Röss, Kaiser, Polte Partnerschaft
(86) Internationale Anmeldenummer: DE9602341
(87) Internationale Veröffentlichungsnummer: WO9720485

(56) Entgegenhaltungen:
- DE-A- 3 101 277
- DE-B- 1 258 020
- DE-C- 473 604
- US-A- 4 977 634

## Beschreibung

Die Erfindung betrifft Ruhe- und Entspannungshilfsmittel, wie Kissen, Bettdecken und allgemein Bettzeug, aber auch Stoff- bzw. Kuscheltiere, wie sie insbesondere Kleinkindern zum Erleichtern des Einschlafens ins Bett gelegt werden.

Um insbesondere bei Schlafstörungen das Einschlafen zu erleichtern sind beispielsweise aus der DE-OS 31 01 277 und der DE-OS 31 40 931 sogenannte Kräuterkissen bekannt. Diese Kräuterkissen umfassen eine allseitig geschlossene und luftdurchlässige Hülle, in die eine Kräutermischung mit Hopfen oder Hopfenextrakt eingefüllt wird. Der Duft der Kräuter soll das Einschlafen erleichtern.

Aus der DE-AS 1 258 020 und DE-PS 473 604 sind Kräuterkissen gemäß dem Oberbegriff des Anspruchs 1 bekannt, die unter anderem Hopfendolden enthalten.

Nachteilig bei diesen bekannten Kräuterkissen und Kräuterunterlagen ist es, daß die abgegebenen und durch den Benutzer eingeatmeten Stoffe kaum definiert und schwer reproduzierbar sind. Dies ist zum einen auf die undefinierten Kräutermischungen und zum anderen auf deren nicht bekannte Wechselwirkungen mit den Hüllen aus verschiedenartigsten Stoffen, wie Roßhaar, Schafwolle, Naturkautschuk, Metallionenabgabeschichten (z. B. Kupfer), usw., zurückzuführen. Damit werden durch den Benutzer solcher bekannter Kräuterkissen neben den gewünschten Kräuteraromastoffen auch andere, in ihrer Menge und Zusammensetzung kaum zu definierende Stoffe eingeatmet. Dies kann häufig zu allergischen Reaktionen führen.

Ein weiterer Nachteil besteht bei den bekannten Kräuterkissen darin, daß sie sich aufgrund ihrer Zusammensetzung aus einer Vielzahl von Stoffen nicht ohne weiteres entsorgen lassen. Auch lassen sich "verbrauchte" Kissen kaum wiederverwenden.

Bei der Verwendung von Hopfen als Kräuterfüllung besteht der weitere Nachteil, daß der Bitterstoff Lupolin, dem die sedidative Wirkung von Hopfen zugeschrieben wird und der auch in Bier enthalten ist, aufgrund seiner Konsistenz die Poren der Hülle verklebt und so die Wirksamkeit des Kräuterkissens vermindert. Derart verklebte Poren begünstigen im Zusammenwirken mit Schweiß auch die Schimmelbildung, was zu einer Gesundheitsgefährdung führen kann.

Es ist daher Aufgabe der vorliegenden Erfindung ein Ruhe und Entspannungshilfsmittel anzugeben, das das Verkleben der Poren der Hülle verhindert.

Die Lösung dieser Aufgabe erfolgt durch die kennzeichenden Merkmale des Anspruchs 1.

Versuche haben ergeben, daß nicht nur der Hopfenbitterstoff Lupolin, sondern auch die insbesondere in den Dolden von Aromahopfen enthaltenen ätherischen Öle eine sedidative Wirkung haben. Den Aromahopfendolden wird das Lupolin entzogen und diese lupolinarmen Aromahopfendolden mit sehr hohem Anteil an ätherischen Ölen werden als Füllstoff verwendet. Durch die Verwendung von lupolinarmen Aromahopfendolden als Füllstoff wird daher Lupolin vermieden und damit verhindert, daß die Poren der Hülle verkleben, gleichzeitig bleibt jedoch die gewünschte sedidative Wirkung erhalten. Durch lupolinarme Aromahopfendolden als Füllstoff wird die Gefahr des Verklebens der Poren der Hülle durch Lupolin erheblich verringert. Durch die Verwendung von reinsten Aromahopfendolden in einer luftdurchlässigen Hülle wird eine genaue Dosierbarkeit und Reproduzierbarkeit der sedidativen Wirkung des erfindungsgemäßen Ruhe- und Entspannungshilfsmittels erreicht. Da als Füllstoff ausschließlich lupolinarme Aromahopfendolden, gegebenenfalls zusammen mit chemisch neutralem Füllmaterial, verwendet werden, gibt es keine Wechselwirkungen mit anderen Inhaltsstoffen. Hierdurch wird ebenfalls die Dosierbarkeit und Reproduzierbarkeit der sedidativen Wirkung des erfindungsgemäßen Ruhe- und Entspannungshilfsmittels gefördert.

Das Vorsehen einer Hülle aus neutralem und staubdichtem Material (Anspruch 2) hat zusätzlich den Vorteil, daß Staub und andere feste Bestandteile der getrockneten Hopfendolden zurückgehalten werden. Folglich gelangen nur die ätherischen Öle in die Atemwege des Benutzers der Erfindung. Damit können auch Allergiker das erfindungsgemäße Ruhe- und Entspannungshilfsmittel nutzen, da gewährleistet ist, daß nur die ätherischen Öle in die Atemwege gelangen und keine Staubteilchen, die zu allergischen Reaktionen führen könnten.

Durch die staubdichte Hülle verlängert sich die Gebrauchsdauer der Erfindung. Bei längerem Gebrauch kommt es zu einer erhöhten mechanischen Beanspruchung der getrockneten Aromahopfendolden und folglich zu einer vermehrten Bildung von Kleinteilen und Staub. Durch die staubdichte Hülle wird verhindert, daß diese Kleinteile der Hopfendolden die Hülle durchdringen und in die Atemwege gelangen.

Staubdichte Hüllen sind engmaschiger gewebt und können daher leichter verkleben. Bei Verwendung von "normalem" Hopfen als Füllstoff verringert sich daher die Gebrauchsdauer. Erst der lupolinarme Füllstoff erlaubt es die Hülle staubdicht und damit engmaschiger zu machen, ohne dadurch die Gebrauchsdauer zu verkürzen.

Durch die Verwendung von reinsten Aromahopfendolden in einer staubdichten, aber luftdurchlässigen Hülle wird eine genaue Dosierbarkeit und Reproduzierbarkeit der sedidativen Wirkung des erfindungsgemäßen Ruhe- und Entspannungshilfsmittels erreicht. Durch die Verwendung von lupolinarmen Aromahopfendolden in einer neutralen, naturbelassenen Hülle wird erreicht, daß nur die ätherischen Öle in die Atemwege des Benutzers gelangen. Da die Hülle neutral ist, gibt es keine Wechselwirkungen zwischen den Materialien der Hülle und den gewünschten Wirkstoffen, den ätherischen Ölen der Aromahopfendolden.

Durch die Verwendung von reinstem, ungebleichten und naturbelassenen Baumwollinlet als Hülle wird die geforderte Geruchs- und Ausgasungsneutralität der Hülle auf einfache Weise erreicht. Durch eine kontrollierte Webart bzw. durch eine entsprechend engmaschige Webung wird die Staubdichtheit erreicht und gleichzeitig bleibt die Luftdurchlässigkeit erhalten (Anspruch 3). Anstelle von Baumwolle kann auch naturbelassenes Leinen verwendet werden.

Durch einen feuchtigkeitsaufnehmenden Überzug wird verhindert, daß Schweiß durch die Hülle an die Aromahopfenfüllung gelangen kann, was zu einer Beeinträchtigung der Wirkung führen würde (Anspruch 4). Durch einen Überzug aus langfaserigem Frottee wird zusätzlich ein Aromaspeicher bereitgestellt. Der Frotteeüberzug wirkt als Pufferspeicher für das Hopfenarome bzw. für die ätherischen Öle und vergleichmäßigt die Abgabe der ätherischen Öle an den Benutzer der Erfindung (Anspruch 5).

Durch die vorteilhafte Ausgestaltung der Erfindung nach Anspruch 6 besteht die Füllung ausschließlich aus Aromahopfendolden ohne jegliche Zusatzstoffe, so daß allergische Reaktionen noch sicherer ausgeschlossen werden.

Gemäß der vorteilhaften Ausgestaltung nach Anspruch 7 besitzt die Hülle oder vorzugsweise der Überzug die Form eines Kissens, so daß das erfindungsgemäße Ruhe- und Entspannungshilfsmittel als Kopfkissen, als Handkissen, als Reisekissen, usw. verwendet werden kann. Besonders vorteilhaft ist auch die Ausgestaltung in Form einer Nackenrolle.

Gemäß der vorteilhaften Ausgestaltung der Erfindung nach Anspruch 8 besitzt die Hülle oder vorzugsweise der Überzug eine flächige Form, so daß sie als Decke, beispielsweise als Zudecke verwendet werden kann. Durch die Verwendung als Decke wird gewährleistet, daß im gesamten Bereich des Bettes das Hopfenaroma vorherrscht. Die flächige Form einer Decke schließt auch die Ausgestaltung als Matrazenauflage ein.

Gemäß einer besonders bevorzugten Ausführungsform nach Anspruch 9 besteht das Füllmaterial von Stofftieren oder Kuscheltieren, wie sie kleine Kinder gerne haben, zumindest teilweise Aromahopfendolden. Dadurch wird das Einschlafen von kleinen Kindern erleichtert.

Gemäß einer weiteren Ausführungsform nach Anspruch 10 besitzt die Hülle die Form einer Atemmaske. Hierdurch wird immer gewährleistet, daß sich die Nase des Ruhe und Entspannung Suchenden im Bereich des Hopfenaromas befindet.

Gemäß den bevorzugten Ausgestaltungen der Erfindung nach Anspruch 12 und 13 werden Kissen etc. bereitgestellt, deren Aromaabgabe entsprechend dem gewünschten Effekt dosiert wird. Zusätzlich kann die abgegebene Dosis auch durch die Dichtheit der Hülle beeinflußt werden. Die angegebenen Werte beziehen sich auf eine staubdichte Hülle mit einer Gewebedichte von 92,3% und einer Lufdurchlässigkeit von 15 l/dm² min nach DIN 53887 bei 200 Pa.

Durch mechanische Extraktion, wie Ausblasen mit Luft, Sieben bzw. Reitern, etc. wird der Anteil an Lupolin verringert und somit die Gefahr des Verklebens der Poren der Hülle noch geringer (Anspruch 14).

Durch eine kontrollierte Niedertemperaturtrocknung wird erreicht, daß der Anteil der ätherischen Öle, die bei der Trocknung ausgasen, minimal gehalten wird (Anspruch 15).

Gemäß der vorteilhaften Ausgestaltung der Erfindung nach Anspruch 16 werden nur Aromahopfendolden verwendet, die aus kontrolliertem Anbau stammen. Aromahopfendolden aus kontrolliertem Anbau sind nahezu frei von Pflanzenschutzrückständen, so daß nur die gewünschten ätherischen Öle ausströmen können. Dadurch wird zusätzlich die Reproduzierbarkeit der sedidativen Wirkung erhöht, da keine Wechselwirkungen zwischen dem Aromahopfen und unerwünschten Zusatzstoffen auftreten können. Zusätzlich wird die Eignung der Erfindung für Allergiker weiter gefördert.

Weitere Einzelheiten, Merkmale und Vorteile der Erfindung ergeben sich aus der nachfolgenden Beschreibung anhand der Zeichnung.

Es zeigt:
- Fig. 1: eine erste Ausführungsform der Erfindung in Form eines Kopfkissens,
- Fig. 2: eine zweite Ausführungsform der Erfindung in Form eines Kuscheltiers,
- Fig. 3: eine schematische Schnittdarstellung einer dritten Ausführungsform der Erfindung mit Aromahopfendolden, Hülle und Überzug, und
- Fig. 4: eine vierte Ausführungsform der Erfindung in Form einer Atemmaske

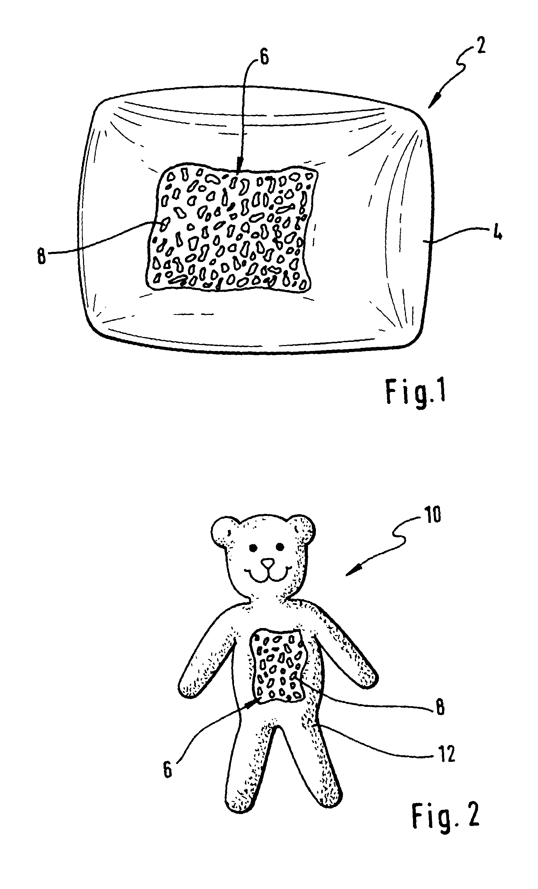

Fig. 1 zeigt eine erste Ausführungsform der Erfindung in Form eines Kissens 2. Das Kissen 2 besitzt eine neutrale, luftdurchlässige Hülle 4, insbesondere aus staubdichtem, naturbelassenem Baumwollstoff oder Leinen. Die Hülle 4 umschließt Füllmaterial 6 in Form von Aromahopfendolden 8, d. h. das Füllmaterial besteht ausschließlich aus Aromahopfendolden 8, Die natürliche bauschige Form der Aromahopfendolden 8 ist ein ideales Füllmaterial für Kissen. Die Hülle 4 umfaßt einen nicht näher dargestellten Verschluß, so daß das Füllmaterial 6 bzw. die Aromahopfendolden 8 leichter von Zeit zu Zeit auswechseln lassen. Vorzugsweise sind die Aromahopfendolden 8 staubdicht in die Hülle 6 eingenäht.

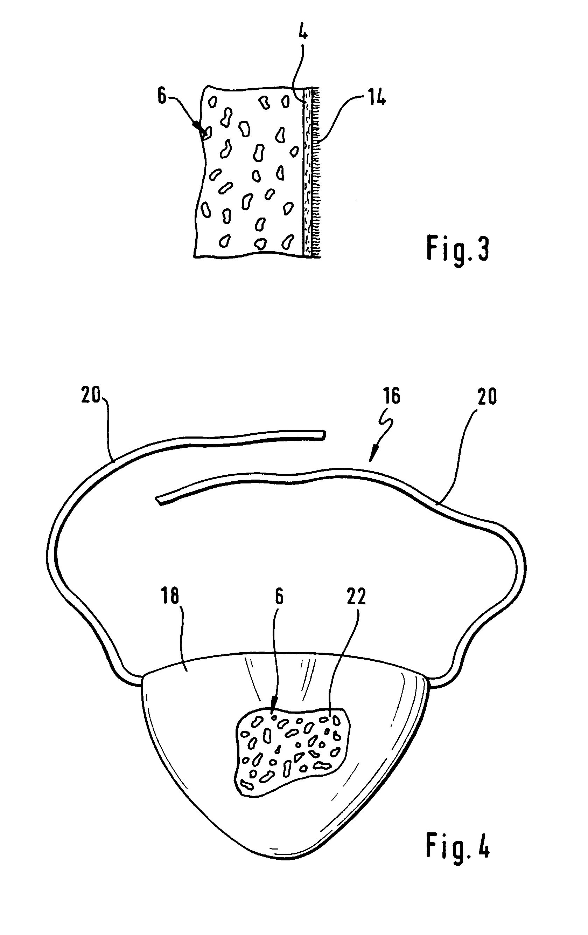

Fig. 2 zeigt eine zweite Ausführungsform der Erfindung in Form eines Kuschel- bzw. Stofftieres 10. Das Stofftier 10 weist einen luftdurchlässigen Überzug 12 auf, der die entsprechende Formgebung bewirkt, in Fig. 2 die Form eines Teddybären. Der Überzug 12 umschließt die Hülle 4 aus dem staubdichten und naturbelassenen Baumwollstoff in der die Aromahopfendolden 6 als Füllmaterial eingeschlossenen sind, so daß sich eine Schichtenfolge ergibt, wie dies in Fig. 3 dargestellt ist. Das Aromahopfendolden 6 können fest in das Stofftier 10 bzw den Überzug 12 eingenäht sein, vorzugsweise weist der Überzug 12 jedoch einen nicht näher dargestellten Verschluß auf, z. B. einen Reißverschluß, so daß die Aromahopfendolen 6 von Zeit zu Zeit ausgewechselt werden können.

Bei den Ausführungsformen nach Fig. 1 und 2 kann das Füllmaterial 6 alternativ auch aus einer Mischung von Aromahopfendolden und herkömmlichen Füllmaterial, wie Federn Holzwolle usw., bestehen. Weiter ist es auch möglich Aromahopfendolden in kleine Taschen oder Säckchen zu verpacken, die dann dem Füllmaterial als Einlage zugegeben werden. Diese Einlagen aromatisieren dann das gesamte Füllmaterial.

Das in Fig. 1 dargestellte Kissen 2 ist vorzugsweise von einem Überzug 14 umgeben, wie dies in Fig. 3 dargestellt ist. Der Überzug 14 besitzt einen Überwurfverschluß, der den Austausch des Überzugs 14 vereinfacht. Der Überzug 14 besteht aus langfaserigem Baumwollfrotte, der zum einen zum Aufnehmen von Feuchtigkeit, insbesondere Schweiß, dient und zum anderen als "Aromapuffer" bzw. als Aromazwischenspeicher funktioniert.

Fig. 4 zeigt eine vierte beispielhafte Ausführungsform der Erfindung in Form einer Atemmaske 16. Die Atemmaske umfaßt ein Mund/Naseteil 18, das mit Bändern 20 am Kopf befestigt wird. Das Mund/Naseteil 18 besitzt staubdichte Taschen oder Hohlräume 22 aus staubdichtem und naturbelassenem Baufwollinlett, in das die Aromahopfendolden 6 eingeschlossen sind.

Für die verschiedenen Hüllen 4 und 22 wird vorzugsweise reinstes, ungebleichtes, naturbelassenes und staubdichtes Baumwollinlett verwendet, weil dadurch allergische Reaktionen der Nutzer nahezu sicher ausgeschlossen werden. Vorzugsweise wird als Hülle 4 bzw. 22 Baumwollinlett mit folgenden Daten verwendet:

| | |
|---|---|
| Fadenzahl Kette | 54 Fd/cm Garn; Nm 70 |
| Fadenzahl Schuß | 29 Fd/cm Garn; Nm 28 |
| Lufdurchlässigkeit | 15 l/dm²min (nach DIN 53887, 200 Pa) |
| Gewebedichte | 92,3% |
| Bindung | Köper |
| Gewicht | 181 g/m² |

Um allergische Reaktionen zu vermeiden und um die Verträglichkeit und Akzeptanz zu erhöhen, enthält das Füllmaterial 6 als Aromaträger ausschließlich reinsten Aromahopfen, der keinerlei bzw. kaum Bitterstoffe (Lupolin) enthält, wie sie in Bitterhopfen enthalten sind.

Aus den Aromahopfendolden 6, die ohnehin schon sehr wenig Lupolin enthalten, wird Lupolin zusaätzlich noch mechanisch extrahiert, bevor die Aromahopfendolden 6 in die Hülle 4 bzw. 22 eingefüllt werden. Hierzu werden die getrockneten Aromahopfendolden zunächst mit Luft durchblasen, so daß lose Staub- und auch Lupolinteilchen entfernt werden. Anschließend werden die so vorbehandelten Aromahopfendolden in einem Sieb gerüttelt, so daß Lupolinteilchen ausgesiebt werden. Vorzugsweise werden die derart behandelten Aromahopfendolden nochmals mit Luft durchblasen, um lose Staub- und Lupolinteilchen zu entfernen.

Als Füllstoff werden insbesondere Aromahopfendolden verwendet, die aus kontrolliertem Anbau stammen. Hierdurch wird gewährleistet, daß die in der Rückstands-Höchstmengenverordnung für Hopfen festgelegten Grenzwerte für Pflanzenschutzmittel vorgegebenen Höchst- und Grenzwerte eingehalten bzw. unterschritten werden.

## Patentansprüche

1. Ruhe- und Entspannungshilfsmittel mit einer luftdurchlässigen Hülle (4; 12; 22), die Füllmaterial (6) mit Hopfen allseitig umhüllt, dadurch gekennzeichnet, daß das Füllmaterial (6) ausschließlich lupolinarme Aromahopfendolden (8; 14; 24) enthält.

2. Ruhe- und Entspannungshilfsmittel nach Anspruch 1, dadurch gekennzeichnet, daß die Hülle (4; 12; 22) aus einem neutralen, staubdichten Material besteht.

3. Ruhe- und Entspannungshilfsmittel nach Anspruch 2, dadurch gekennzeichnet, daß die Hülle (4; 12; 22) aus reinstem, naturbelassenem und staubdichtem Baumwollinletstoff besteht.

4. Ruhe- und Entspannungshilfsmittel nach wenigstens einem der vorhergehenden Ansprüche, gekennzeichnet durch einen Überzug aus feuchtigkeitsaufnehmenden Material.

5. Ruhe- und Entspannungshilfsmittel nach Anspruch 4, dadurch gekennzeichnet, daß der Überzug aus langfaserigem Baumwollfrottee besteht.

6. Ruhe- und Entspannungshilfsmittel nach wenigstens einem der vorhergehenden Ansprüche, dadurch gekennzeichnet, daß das Füllmaterial (6) vollständig aus Aromahopfendolden (8) besteht.

7. Ruhe- und Entspannungshilfsmittel nach wenigstens einem der vorhergehenden Ansprüche, dadurch gekennzeichnet, daß die Hülle (4) und/oder der Überzug die Form eines Kissens (2) aufweist.

8. Ruhe- und Entspannungshilfsmittel nach wenigstens einem der vorhergehenden Ansprüche 1 bis 6, dadurch gekennzeichnet, daß die Hülle und/oder der Überzug die Form einer Decke aufweist.

9. Ruhe- und Entspannungshilfsmittel nach wenigstens einem der vorhergehenden Ansprüche 1 bis 6, dadurch gekennzeichnet, daß die Hülle (12) und/oder der Überzug die Form eines Stofftieres (10) aufweist.

10. Ruhe- und Entspannungshilfsmittel nach wenigstens einem der vorhergehenden Ansprüche 1 bis 6, dadurch gekennzeichnet, daß die Hülle (22) und/oder der Überzug die Form einer Atemmaske (16) aufweist.

11. Ruhe- und Entspannungshilfsmittel nach Anspruch 10, dadurch gekennzeichnet, daß die Atemmaske (16) Taschen aufweist, die die Aromahopfendolden enthalten.

12. Ruhe- und Entspannungshilfsmittel nach wenigstens einem der vorhergehenden Ansprüche, dadurch gekennzeichnet, daß das Verhältnis von Füllgewicht der Aromahopfendolden zu Ausgasungsfläche der Hülle für beruhigende Wirkung im Bereich zwischen 0,042 und 0,063 g/cm² liegt.

13. Ruhe- und Entspannungshilfsmittel nach wenigstens einem der vorhergehenden Ansprüche, dadurch gekennzeichnet, daß das Verhältnis von Füllgewicht der Aromahopfendolden zu Ausgasungsfläche der Hülle für schlaffördernde Wirkung im Bereich zwischen 0,104 und 0,125 g/cm² liegt.

14. Ruhe- und Entspannungshilfsmittel nach wenigstens einem der vorhergehenden Ansprüche, dadurch gekennzeichnet, daß aus den Aromahopfendolden Lupolin mechanisch extrahiert wird.

15. Ruhe- und Entspannungshilfsmittel nach wenigstens einem der vorhergehenden Ansprüche, dadurch gekennzeichnet, daß die Aromahopfendolden mittels einer kontrollierten Niedertemperaturtrocknung getrocknet sind.

16. Ruhe- und Entspannungshilfsmittel nach wenigstens einem der vorhergehenden Ansprüche, dadurch gekennzeichnet, daß die Aromahopfendolden aus kontrolliertem Anbau stammen.

## Claims

1. Resting and relaxing aid comprising an air-permeable covering (4; 12; 22) which encloses a stuffing (6) comprising hops on all sides, characterised in that said stuffing (6) exclusively contains aromatic hops cones (8; 14; 24) low in lupulin.

2. Resting and relaxing aid according to claim 1, characterised in that said covering (4; 12; 22) consists of a neutral, dustproof material.

3. Resting and relaxing aid according to claim 2, characterised in that said covering (4; 12; 22) consists of purest, unadulterated natural, dustproof cotton ticking cloth.

4. Resting and relaxing aid according to at least one of the preceding claims, characterised by a case of humidity-absorbing material.

5. Resting and relaxing aid according to claim 4, characterised in that said case consists of long-fiber cotton terry cloth.

6. Resting and relaxing aid according to at least one of the preceding claims, characterised in that said stuffing material (6) entirely consists of aromatic hops cones (8; 14; 24).

7. Resting and relaxing aid according to at least one of the preceding claims, characterised in that said covering (4) and/or said case has the form of a pillow (2).

8. Resting and relaxing aid according to at least one of the preceding claims 1 to 6, characterised in that said covering and/or said case has the form of a blanket.

9. Resting and relaxing aid according to at least one of the preceding claims 1 to 6, characterised in that said covering (12) and/or said case has the form of a stuffed toy animal (10).

10. Resting and relaxing aid according to at least one of the preceding claims 1 to 6, characterised in that said covering (22) and/or said case has the form of a breathing mask (16).

11. Resting and relaxing aid according to claim 10, characterised in that said breathing mask (16) includes pockets which contain aromatic hops cones.

12. Resting and relaxing aid according to at least one of the preceding claims, characterised in that the ratio of filling weight of said aromatic hops cones to exhalation surface of said covering is situated in the range between 0.042 and 0.063 g/cm² for a calming effect.

13. Resting and relaxing aid according to at least one of the preceding claims, characterised in that the ratio of filling weight of said aromatic hops cones to exhalation surface of said covering is situated in the range between 0.104 and 0.125 g/cm² for a sleep-inducing effect.

14. Resting and relaxing aid according to at least one of the preceding claims, characterised in that lupulin is mechanically extracted from said aromatic hops cones.

15. Resting and relaxing aid according to at least one of the preceding claims, characterised in that said aromatic hops cones are dried by means of controlled low-temperature drying.

16. Resting and relaxing aid according to at least one of the preceding claims, characterised in that said aromatic hops cones originate from controlled cultivation.

## Revendications

1. Objet apaisant et relaxant ayant une enveloppe (4; 12; 22) perméable à l'air qui recouvre complètement une matière de remplissage (6) avec du houblon, caractérisé par le fait que la matière de remplissage (6) contient exclusivement des cônes de houblon aromatique (8; 14; 24) ayant une faible teneur en lupuline.

2. Objet apaisant et relaxant selon la revendication 1, caractérisé par le fait que l'enveloppe (4; 12; 22) est composée d'une matière neutre et étanche à la poussière.

3. Objet apaisant et relaxant selon la revendication 2, caractérisé par le fait que l'enveloppe (4; 12; 22) est composée d'une étoffe pour édredon en coton le plus pur et le plus naturel possible, étanche à la poussière.

4. Objet apaisant et relaxant selon au moins l'une des revendications précédentes, caractérisé par une housse d'un matériau capable d'absorber l'humidité.

5. Objet apaisant et relaxant selon la revendication 4, caractérisé par le fait que la housse est en tissu-éponge de coton peigné.

6. Objet apaisant et relaxant selon au moins l'une des revendications précédentes, caractérisé par le fait que la matière de remplissage (6) est constituée entièrement de cônes de houblon aromatique (8).

7. Objet apaisant et relaxant selon au moins l'une des revendications précédentes, caractérisé par le fait que l'enveloppe (4) et/ou la housse a la forme d'un coussin (2).

8. Objet apaisant et relaxant selon au moins l'une des revendications précédentes 1 à 6, caractérisé par le fait que l'enveloppe et/ou la housse a la forme d'une couverture.

9. Objet apaisant et relaxant selon au moins l'une des revendications précédentes 1 à 6, caractérisé par le fait que l'enveloppe (12) et/ou la housse a la forme d'une peluche en tissu (10).

10. Objet apaisant et relaxant selon au moins l'une des revendications précédentes 1 à 6, caractérisé par le fait que l'enveloppe (22) et/ou la housse a la forme d'un masque respiratoire (16).

11. Objet apaisant et relaxant selon la revendication 10, caractérisé par le fait que le masque respiratoire (16) présente des poches contenant les cônes de houblon aromatique.

12. Objet apaisant et relaxant selon au moins l'une des revendications précédentes, caractérisé par le fait que le rapport entre le poids de remplissage des cônes de houblon aromatique et la surface d'émanation des essences de l'enveloppe est compris entre 0,042 et 0,063 g/cm² pour un effet calmant.

13. Objet apaisant et relaxant selon au moins l'une des revendications précédentes, caractérisé par le fait que le rapport entre le poids de remplissage des cônes de houblon aromatique et la surface d'émanation des essences de l'enveloppe est compris entre 0,104 et 0,125 g/cm² pour un effet favorisant le sommeil.

14. Objet apaisant et relaxant selon au moins l'une des revendications précédentes, caractérisé par le fait que la lupuline est extraite des cônes de houblon aromatique de manière mécanique.

15. Objet apaisant et relaxant selon au moins l'une des revendications précédentes, caractérisé par le fait que les cônes de houblon aromatique sont séchés au moyen d'un séchage controlé à basse température.

16. Objet apaisant et relaxant selon au moins l'une des revendications précédentes, caractérisé par le fait que les cônes de houblon aromatique proviennent de culture contrôlée.
